# EUROPEAN PATENT APPLICATION

(11) **EP 4 450 108 A1**
(43) Date of publication of application: **23.10.2024**
(21) Application number: 23168923.3
(22) Date of filing: 20.04.2023
(51) Int. Cl.: A61M 25/00

(54) **A CATHETER FOR DELIVERING MEDICAL THERAPY TO A REMOTE SITE IN A BODY**

(71) Applicant: IMDS R&D BV, 9301 NZ Roden (NL)
(72) Inventor: SCHULTING, Edwin Alexander, 9301 NZ Roden (NL)
(74) Representative: V.O.

(57) **Abstract**

A tubular catheter wall of a catheter (1) comprises a randomized skeleton (7) having a microfabricated cut pattern (7A) with an overall skeleton cut-away ratio between 40% and 90%. The randomized skeleton (7), which is arranged between an inner liner and an outer laminate, comprises a plurality of interconnected struts (21L-28L, 31L-38L, 21R-28R, 31R-38R). Each strut has a corresponding pair of two mutually opposing longitudinal strut edges (41, 42). Each strut edge (41, 42) has random bends and/or random cusps, which makes each strut unique. The randomly shaped skeleton strut edges not only provide improved mutual bondings between the skeleton, inner liner and outer laminate, but also provide improved torquability of the catheter, including a preventive effect on the occurrence of the whip phenomenon during torquing.

## Description

The invention relates to a catheter for delivering medical therapy to a remote site in a body.

In said medical therapy, such as for example an interventional cardiovascular or neurovascular application, such a catheter must be angled through the tortuous bends and curves of a (vasculature) passageway to arrive at the targeted anatomy. Such a catheter requires sufficient flexibility, particularly closer to its distal end, to navigate such tortuous pathways. However, other design aspects must also be considered. For example, the catheter must also be able to provide sufficient torquability (i.e., the ability to transmit torque applied at the proximal end all the way to the distal end), pushability (i.e., the ability to transmit axial push to the distal end), and structural integrity for performing intended medical functions.

For example, with respect to the above-mentioned torquability it often happens in interventional cardiovascular or neurovascular applications that catheters have a preferred orientation in a certain arterial tree. This is due to interactions between the configurations of the catheter and the arterial tree. As a result thereof it is sometimes very difficult or impossible for the medical practitioner to torque the catheter from outside the patient-body at the proximal end of the catheter in such manner that the tip of the catheter is rotated and directed to the origin of the targeted branched passageway. The reason is that the preferred orientation of the catheter relative to a certain blood vessel structure in combination with the known phenomenon called "whip" in many cases causes that torquing of the catheter practically all the time results into one or more exact 360 degrees rotations of the distal tip. This way the distal tip practically always ends up into its erroneous starting orientation, which is not directed to the origin of the targeted branched passageway. In such cases said whip phenomenon is usually caused by the torquing of the catheter from outside the patient-body, which is far away from the distal tip. As torque builds during torquing, energy is stored in the catheter. And when the catheter ultimately and suddenly rotates, such rotation is caused by the fact that a certain uncontrollable amount of the stored energy is suddenly released. In fact, the catheter does not use all the stored energy to automatically arrive at its preferred orientation relative to the blood vessel structure again. Instead, the remaining unused part of the stored energy is not enough to overcome the resistance from the stable support of the catheter within the blood vessel structure in the catheter's preferred orientation, in which the catheter's distal tip is not directed to the origin of the targeted branched passageway. Clearly, this is a frustrating experience for medical practitioners.

It is an object of the present invention to provide a new catheter design, which not only offers additional design freedom, but also improves the catheter's torquability, inter alia by providing a preventive effect on the occurrence of the above-mentioned whip phenomenon during torquing.

For that purpose the invention provides a catheter according to the appended independent claim 1. Preferable embodiments of the invention are provided by the appended dependent claims 2-9.

Hence, the invention provides a catheter for delivering medical therapy to a remote site in a body, wherein:
the catheter has a proximal end, a distal end and a longitudinal direction extending from the proximal end to the distal end;
the catheter comprises a tubular catheter wall, which is surrounding a lumen structure of the catheter, and which is extending from the distal end, proximally along the longitudinal direction, to at least 100 mm proximal from the distal end;
the catheter wall comprises a randomized-skeleton wall section, which is extending in a randomized-skeleton range along the longitudinal direction, wherein said randomized-skeleton range is at least extending between 10 mm proximal from the distal end and 100 mm proximal from the distal end;
the randomized-skeleton wall section comprises:
   - a randomized skeleton, which is extending at least all along said randomized-skeleton range, and which provides reinforcement of the catheter wall in the longitudinal direction, as well in a circumferential direction around the longitudinal direction, wherein the randomized skeleton is an interrupted tubular reinforcement wall obtained by microfabricating a cut pattern in a fully uninterrupted tubular reinforcement wall made of a reinforcement material,
   - an inner liner, which is co-axially surrounded by said randomized skeleton relative to a center line of the catheter wall, and
   - an outer laminate, which is co-axially surrounding said randomized skeleton relative to the center line of the catheter wall;
the randomized skeleton comprises a plurality of interconnected struts, wherein, as seen in radial view on the randomized skeleton, said radial view being radially in a cylindrical coordinate system relative to the center line:
   - each of said struts has a corresponding pair of two mutually opposing longitudinal strut edges alongside one another, which are determining a corresponding distribution of tangential thickness of a strut concerned longitudinally along the strut concerned, said tangential thickness being tangentially in a cylindrical coordinate system relative to the center line,
   - said longitudinal strut edges are bordering said cut pattern, and
   - each of said longitudinal strut edges has random bends and/or random cusps;
an overall skeleton cut-away ratio of a longitudinal section of the catheter wall in a longitudinal range along the longitudinal direction is defined as the percentage of:
   - the volume of all parts, within said longitudinal range, of said reinforcement material being cut-away from said fully uninterrupted tubular reinforcement wall according to said microfabricated cut pattern,
      relative to
   - the volume of all parts, within said longitudinal range, of said reinforcement material of said fully uninterrupted tubular reinforcement wall; and
the overall skeleton cut-away ratio of the randomized-skeleton wall section in said randomized-skeleton range is between 40% and 90%.

In the above-recited features of the present invention, key features of the invention are:
i. the random bends and/or random cusps of each of the two mutually opposing longitudinal strut edges of each strut of the plurality of interconnected struts of the randomized skeleton of the catheter wall; and
ii. the overall skeleton cut-away ratio of the randomized-skeleton wall section being between 40% and 90%.

Said key feature (i) relates to the random bends and/or random cusps of the randomized skeleton in the randomized-skeleton range along the longitudinal direction. As compared to struts of known skeletons, which have skeleton strut edges shaped as repeated patterns of regular lines or regular curves, the randomly shaped skeleton strut edges of the present invention provide improved mutual contact surfaces, and hence improved mutual bondings, between the skeleton material, the material of the inner liner and the material of the outer laminate. Said improved mutual bondings provides additional design freedom to make catheters more sophisticated to meet increasing design challenges to enable more advanced medical therapies. For example, as compared to catheters having known skeletons, said improved mutual bondings provided by the randomized skeleton of a catheter according to the invention allows for designing the randomized-skeleton wall section with smaller radial thickness without sacrificing on the catheter's strength.

Additionally, the randomly shaped skeleton strut edges of the present invention have a preventive effect on the occurrence of the above-mentioned whip phenomenon, which occurs when a catheter assumes preferred orientations in certain arterial trees. Thanks to the random shapes embodied in a catheter according to the invention, the internal stress distribution within the randomized-skeleton wall section of the catheter changes more randomly, in fact more as in natural structures, in response to the catheter being moved and torqued by a physician within an arterial tree, as compared to a known catheter having skeleton strut edges which are shaped with repeated patterns of regular lines or curves, which is in fact unnatural. In fact, every single strut of the randomized skeleton of a catheter according to the invention has a unique shape. It is to be noted that natural vessels of a body in fact do have randomly shaped structures as well, i.e. naturally grown structures. When a physician is moving and torquing a catheter according to the invention within a natural vessel structure, the more randomly changing internal stress distribution within the randomized-skeleton wall section of the catheter results in a more gradual rotational adaptation behaviour of the catheter to the random characteristics of the natural vessel structure and thereby contributes to the prevention of occurrences of said preferred orientations of a catheter in the natural vessel structure. In other words, the invention provides more natural interaction between catheter and vessel structures of a body when the catheter is moved and torqued in said vessel structures.

Said key feature (ii) relating to the overall skeleton cut-away ratio of the randomized-skeleton wall section being between 40% and 90%, allows for a good flexibility of the catheter in the randomized-skeleton range, which allows for navigating challenging tortuous pathways.

Furthermore, it will be readily appreciated that the randomized skeleton provides the randomized-skeleton wall section with good pushability and integrity of lumen structure. Other longitudinal sections of the catheter can be designed in many various ways and be connected firmly to, or integrally with, the randomized-skeleton wall section having the randomized skeleton. Hence, the invention allows the catheter to be reliably made in an efficient manner, and to be designed with favourable properties with regard to flexibility, torquability, pushability and integrity of lumen structure.

It is noted that manufacturing said high overall skeleton cut-away ratio (at least 40%) of the randomized-skeleton wall section according to the invention requires inventive measures to solve several problems. The reason is that the inner liner of a catheter having a skeleton for many applications typically is a tube made of, for example, PTFE (polytetrafluoroethylene, e.g. Teflon^{®}), PVDF (polyvinylidene fluoride, e.g. Kynar^{®}) or HDPE (high-density polyethylene), which during manufacturing of the catheter is axially inserted into the skeleton, and which is then expanded against the skeleton by inflation and heat. For said high cut-away ratios the problems arise that the tube made of, for example, PTFE, PVDF or HDPE being inflated against the skeleton will bulge out of the large cut-outs in the skeleton material and/or will break under the inflation pressure. Another problem, especially for skeletons made of less strong material, is that the skeleton may severely deform and/or crack and/or break under the inflation pressure due to the fact that the skeleton is severely weakened by the large cut-outs in the skeleton material.

In connection therewith, the present invention inter alia is based on the new insights, as disclosed herein, that said problems can be solved by introducing, temporarily during the manufacturing of a catheter according to the invention, a heat-shrinkable outer tube over the outer side of the randomized skeleton, so that the heat-shrinkable outer tube temporarily closes off the cut-outs in the skeleton material during said expansion of the inner liner by inflation and heat, while at the same time the heat-shrinkable outer tube temporarily reinforces the randomized skeleton during said expansion of the inner liner by inflation and heat. After the inner liner has been successfully expanded against the skeleton and after the catheter under construction has been cooled, the temporary heat-shrinkable outer tube can be removed from the outer side of the skeleton, and be replaced by the final outer laminate over the outer side of the skeleton.

Alternatively, another new insight, as disclosed herein, is that said problems can be solved by performing the following manufacturing steps for assembling the inner liner and the outer laminate to the skeleton in case of said high overall skeleton cut-away ratio (at least 40%) of the randomized-skeleton wall section of the catheter wall of a catheter according to the invention. Firstly, a tubular base layer of fluorinated ethylene propylene (FEP) is extruded and the inner liner is extruded over the base layer. Next, the FEP base layer together with the inner liner is axially inserted into the skeleton. Next, the outer laminate is slided over the outer side of the skeleton. Thereafter, a temporary heat-shrinkable outer FEP tube is slided over the outer laminate, and the total assembly is heated. During heating the temporary heat-shrinkable outer FEP tube provides high compression onto the assembly of the inner liner, the skeleton and the outer laminate. After the catheter under construction has been cooled, the temporary heat-shrinkable outer tube can be removed from the outer side of the skeleton, and the FEP base layer can be axially pulled out of the assembly, making use of the favourable properties of FEP with respect to being pulled. Thus, a very close fit of the inner liner against the inner side of the skeleton is obtainable.

It is noted that the last-mentioned use of such an extruded FEP base layer is a more effective and much less expensive alternative as compared to the traditional use of a temporary silver plated copper wire, which at the end of a manufacturing sub-process is axially pulled out of an inner liner of a catheter under construction.

Another advantage of the present invention is that, thanks to the high overall skeleton cut-away ratio (at least 40%) of the randomized-skeleton wall section of the catheter wall of a catheter according to the invention, and thanks to the above-mentioned insights as disclosed herein, the invention allows for efficiently embedding a radiopaque element (e.g. a radiopaque marker) in the catheter wall, since the radiopaque element, can be perfectly located in a space of the cut pattern, said space being bounded by the randomized skeleton, the inner layer and the outer laminate.

In a preferable embodiment of a catheter according to the invention, said randomized-skeleton range is at least extending between 0.5 mm proximal from the distal end and 100 mm proximal from the distal end. Thus the randomized skeleton is distally extending yet closer to the distal end.

More preferably, said randomized-skeleton range is at least extending between the distal end and 100 mm proximal from the distal end.

In another preferable embodiment of a catheter according to the invention, the overall skeleton cut-away ratio of the randomized-skeleton wall section in said randomized-skeleton range is between 60% and 85%.

In another preferable embodiment of a catheter according to the invention, a radiopaque element is embedded in the catheter wall in that the radiopaque element is located in a space of the cut pattern, said space being bounded by the randomized skeleton, the inner layer and the outer laminate.

The catheter according to the invention may be embodied as a rapid exchange catheter having the tubular catheter wall as a distal rapid exchange tubular segment which is spaced from the proximal end of the rapid exchange catheter.

Such a rapid exchange catheter has a proximal catheter segment, proximally from the distal rapid exchange tubular segment, which may, for example, be a push rod which is connected to the distal rapid exchange tubular segment. Such a push rod may advantageously have a circular transverse cross section with a diameter ≤ 0.40 mm to support 1:1 torquability from the proximal end to the distal end. Such a 1:1 torquability is highly desirable for this kind of device as the physician needs to be able to precisely steer the catheter towards a target site, such as a bend or a side branch of a vasculature passageway.

In another preferable embodiment of a catheter according to the invention, the tubular catheter wall being said distal rapid exchange tubular segment of said rapid exchange catheter comprises at least one perfusion side hole, which is extending from the lumen structure, via successive interruptions of the inner liner, the randomized skeleton and the outer laminate, to the radially outwards exterior environment of the tubular catheter wall for enabling perfusion flow transversely through the tubular catheter wall from the lumen structure to said exterior environment.

One such perfusion side hole, or preferably a plurality of such perfusion side holes, may be desirable when a distal rapid exchange tubular segment of a rapid exchange catheter is, for example, deeply intubated into coronary arteries during Percutaneous Coronary Intervention. Without such perfusion side hole(s) the distal rapid exchange tubular segment might strongly reduce/block the distal coronary flow and might so cause procedural induced ischemia.

An advantageous arrangement of such perfusion side holes could, for example, be nine perfusion side holes placed with equal angular spacing along a spiral path which is extending between 40 mm proximal from the distal end to 85 mm proximal from the distal end of the distal rapid exchange tubular segment. Therein, each of the nine perfusion side holes can be surrounded by a corresponding set of mutually connected ones of the randomized skeleton's struts having the random bends and/or random cusps. Preferably, the perfusion side holes should have a hole diameter small enough (for example ≤ 0.30 mm) to prevent that the tip of a guide wire inside the rapid exchange tubular segment could stick out of such a perfusion hole and cause vessel perforation.

Alternatively, the catheter according to the invention may be embodied as an over-the-wire (OTW) catheter, wherein the tubular catheter wall and the lumen structure of the catheter are extending all along the longitudinal direction from the proximal end to the distal end.

Such an over-the-wire catheter may advantageously have an over-the-wire catheter skeleton, which is extending all along the longitudinal direction from the proximal end to the distal end, and which provides reinforcement of the catheter wall in the longitudinal direction, as well in the circumferential direction around the longitudinal direction, wherein the over-the-wire catheter skeleton is an interrupted tubular reinforcement wall obtained by microfabricating a cut pattern in a fully uninterrupted tubular reinforcement wall made of a reinforcement material. In this case, the randomized skeleton of the randomized-skeleton wall section may be only a section of the over-the-wire catheter skeleton, or may even be extending all along the longitudinal direction from the proximal end to the distal end. Preferably the over-the-wire catheter skeleton is designed to support 1:1 torquability from the proximal end to the distal end of the over-the-wire catheter. Alternatively to said over-the wire skeleton, the over-the-wire catheter may, in longitudinal sections outside the randomized-skeleton range of the randomized skeleton, also be designed with, for example, braids or wire coil structures to support the 1:1 torquability.

In another preferable embodiment of a catheter according to the invention, the tubular catheter wall of said over-the-wire (OTW) catheter comprises at least one perfusion side hole, which is extending from the lumen structure, via successive interruptions of the inner liner, the randomized skeleton and the outer laminate, to the radially outwards exterior environment of the tubular catheter wall for enabling perfusion flow transversely through the tubular catheter wall from the lumen structure to said exterior environment.

One such perfusion side hole, or preferably a plurality of such perfusion side holes, may be desirable to prevent procedural induced ischemia.

In the following, the invention is further elucidated with reference to a non-limiting embodiment and with reference to the schematic figures in the appended drawing, in which the following is shown.
Fig. 1 shows, in a longitudinal side view, an example of an embodiment of a catheter according to the invention, wherein in Fig. 1 the randomized-skeleton range of the randomized-skeleton wall section of the catheter wall has been indicated by a brace mark.
Fig. 2 shows, in a perspective view, a short longitudinal section of the randomized skeleton of the randomized-skeleton wall section of the catheter of Fig. 1.
Fig. 3A shows a short longitudinal section of the randomized-skeleton wall section of the catheter of Fig. 1, wherein the shown longitudinal section comprises a part of the randomized skeleton, and wherein the shown view is a longitudinal sectional view, which contains the center line of the catheter wall.
Fig. 3B shows a cross-section through the longitudinal section of the randomized-skeleton wall section of Fig. 3A, wherein the shown cross-section is perpendicular to the center line of the catheter wall.
Fig. 4 shows a part of the short longitudinal section of the randomized skeleton of Fig. 2 again, however, wherein this time the shown view is a top view on the longitudinal section in an imaginary "unrolled tube" state of the longitudinal section.
Fig. 5 shows, in a similar manner as in Fig. 3A, another short longitudinal section of the randomized-skeleton wall section of the catheter of Fig. 1, wherein Fig. 5 shows two perfusion side holes of the randomized-skeleton wall section.
Fig. 6A shows an example of an initial Finite Element Analysis (FEA) mesh of a portion of one regular strut of an initial regular skeleton for a catheter, wherein the shown view is similar to the view of Fig. 4 in that the shown view is a top view on the initial FEA mesh in an imaginary "unrolled tube" state of the initial FEA mesh.
Fig. 6B shows, in a view similar to the view of Fig. 6A, an example of a portion of a distorted FEA mesh of a corresponding portion of a distorted strut of a distorted skeleton, said distorted FEA mesh being used in an example of a skeleton design process based on a randomizing algorithm for obtaining the random bends and/or the random cusps of the longitudinal strut edges of the randomized skeleton of a catheter according to the invention.

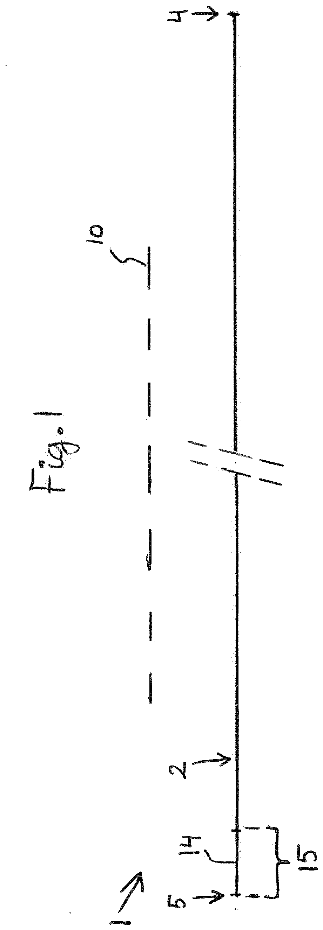

The reference signs used in Figs. 1-5 are referring to the above-mentioned parts and aspects of the invention, as well as to related parts and aspects, in the following manner.
- 1: catheter
- 2: tubular catheter wall
- 3: lumen structure
- 4: proximal end
- 5: distal end
- 7: randomized skeleton
- 7A: cut pattern
- 8: inner liner
- 9: outer laminate
- 10: longitudinal direction
- 11: circumferential direction
- 12: center line
- 14: randomized-skeleton wall section
- 15: randomized-skeleton range
- 21L-28L, 31L-38L: left-handed struts
- 21R-28R, 31R-38R: right-handed struts
- 41, 42: longitudinal strut edges
- 51, 52: perfusion side holes
- 68-85: connection nodes
- 100: initial FEA mesh
- 100A: distorted FEA mesh
- 101-112: nodes of the initial FEA mesh 100
- 103A, 104A, 107A, 108A, 111A, 112A,: distorted nodes of the distorted FEA mesh 100A

Based on the above introductory description, including the brief description of the drawing figures, and based on the above-listed reference signs used in Figs. 1-5, the embodiment of Figs. 1-5 is for the greatest part readily self-explanatory. The following extra explanations are given.

Fig. 1 shows the catheter 1 having the longitudinal direction 10, the proximal end 4 and the distal end 5. Fig. 1 further shows the randomized-skeleton range 15 of the randomized-skeleton wall section 14 of the catheter wall 2.

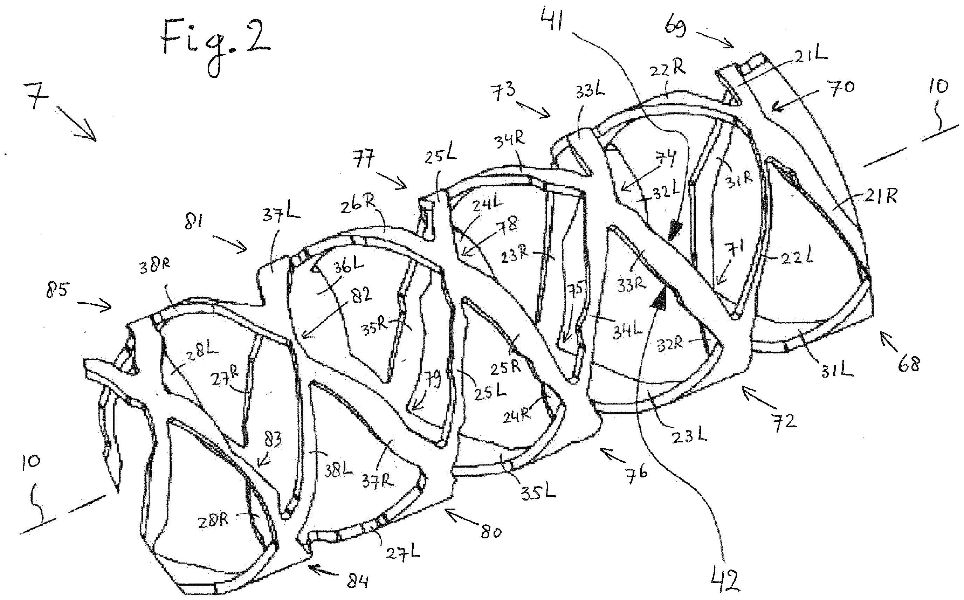

The perspective view of Fig. 2 shows that the randomized skeleton 7 of the randomized-skeleton wall section 14 of the catheter 1 of Fig. 1 has the left-handed struts 21L-28L and 31L-38L, the right-handed struts 21R-28R and 31R-38R, and the connection nodes 68-85.

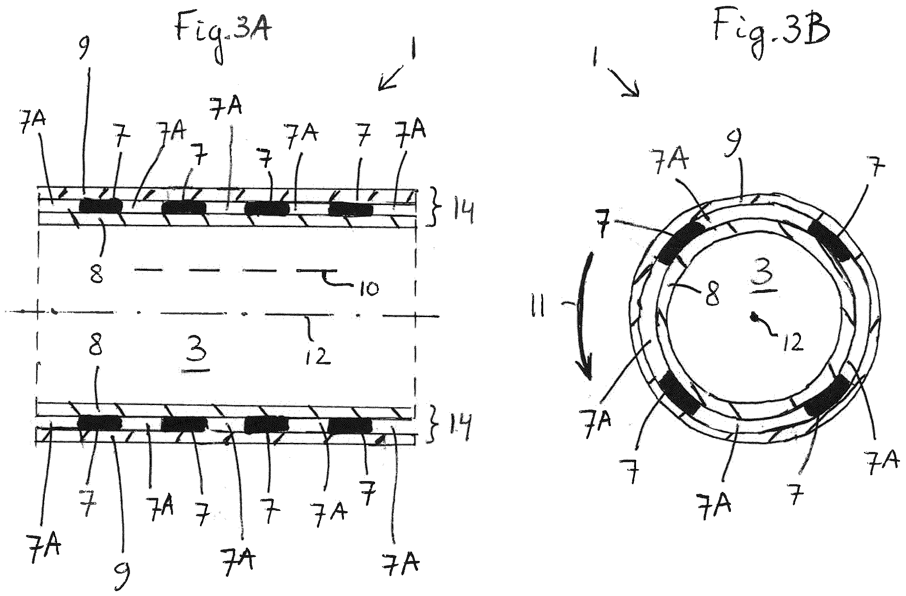

Figs. 3A-3B show that the randomized-skeleton wall section 14 not only has the skeleton 7, which comprises the cut pattern 7A, but also has the inner liner 8 and the outer laminate 9. In the example of Figs. 3A-3B, the lumen structure 3 of the catheter 1 is a single lumen structure. Alternatively, a catheter according to the invention may have a multiple lumen structure.

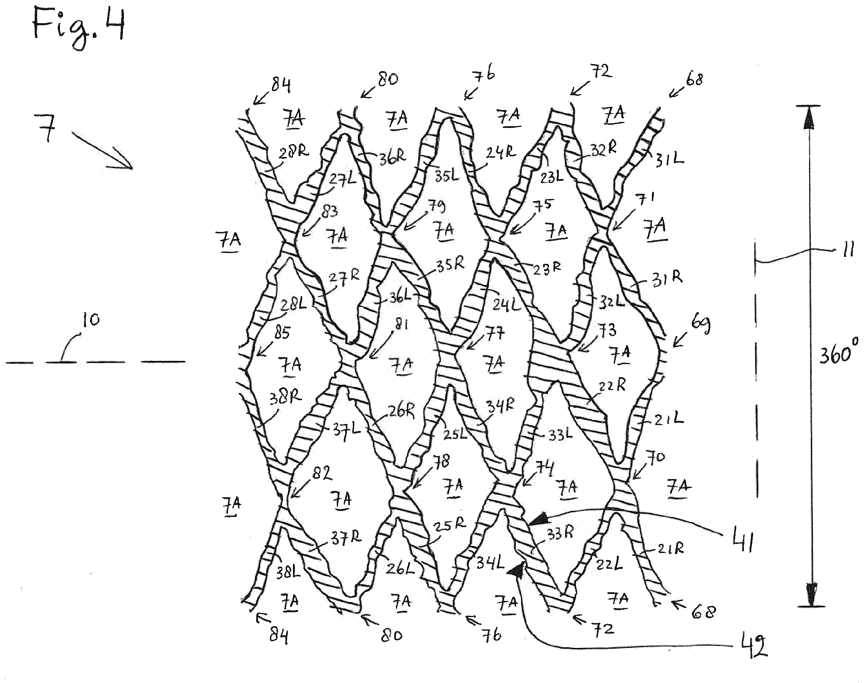

Fig. 4 shows a short longitudinal section of the randomized skeleton 7 of the randomized-skeleton wall section 14 of the catheter 1. In Fig. 4, the shown view is a top view on the longitudinal section in an imaginary "unrolled tube" state of the longitudinal section. To explain the term "unrolled tube" it is noted that the randomized skeleton is a tubular wall (more particularly an interrupted tubular reinforcement wall), and that such a tubular wall imaginarily can be unrolled into a completely straight state by imaginarily unrolling it onto a fully straight plane after an imaginary straight cut, parallel to the center line of the catheter, has been made over the full length of the tubular wall. In Fig. 4 said imaginary straight cut is a straight line that passes through the connection nodes 68, 72, 76, 80 and 84 of the randomized skeleton 7. In Fig. 4 the reference numerals 68, 72, 76, 80 and 84 are shown both in the lower part of Fig. 4 and in the upper part of Fig. 4, since the locations to which these reference numerals 68, 72, 76, 80 and 84 are pointing will join when the completely straight state of Fig. 4 would imaginarily be "rolled up" again to form the tubular state of the randomized skeleton 7 as shown in the perspective view of Fig. 2.

From Figs. 2 and 4 (best seen in Fig. 4) it can be inferred that at each of the connection nodes 70-83 four of the struts are connected with one another, i.e. two of the left-handed struts and two of the right-handed struts. For example, at the connection node 74 the two left-handed struts 33L, 34L and the two right-handed struts 33R, 34R are all interconnected.

From Figs. 2 and 4 (best seen in Fig. 4) it can further be inferred that each one of the shown struts 21L-28L, 31L-38L, 21R-28R and 31R-38R has a corresponding pair of two mutually opposing longitudinal strut edges alongside one another, wherein each of said longitudinal strut edges has random bends and/or random cusps. For simplicity, in Figs. 2 and 4 for only one of the shown struts these longitudinal strut edges have been indicated with reference numerals. See the two mutually opposing longitudinal strut edges 41 and 42 of the right-handed strut 33R. From Figs. 2 and 4 it will be readily appreciated that every single strut of the randomized skeleton 7 of a catheter 1 according to the invention has a unique shape.

In the introduction of the present disclosure it has been mentioned that a catheter according to the invention may be embodied as a rapid exchange catheter having the tubular catheter wall as a distal rapid exchange tubular segment which is spaced from the proximal end of the rapid exchange catheter. Also it has been mentioned that the catheter may alternatively be embodied as an over-the-wire (OTW) catheter, wherein the tubular catheter wall and the lumen structure of the catheter are extending all along the longitudinal direction from the proximal end to the distal end. It has further been mentioned that in both embodiments (rapid exchange catheter or over-the-wire catheter), the tubular catheter wall may comprise at least one perfusion side hole, which is extending from the lumen structure, via successive interruptions of the inner liner, the randomized skeleton and the outer laminate, to the radially outwards exterior environment of the tubular catheter wall for enabling perfusion flow transversely through the tubular catheter wall from the lumen structure to said exterior environment.

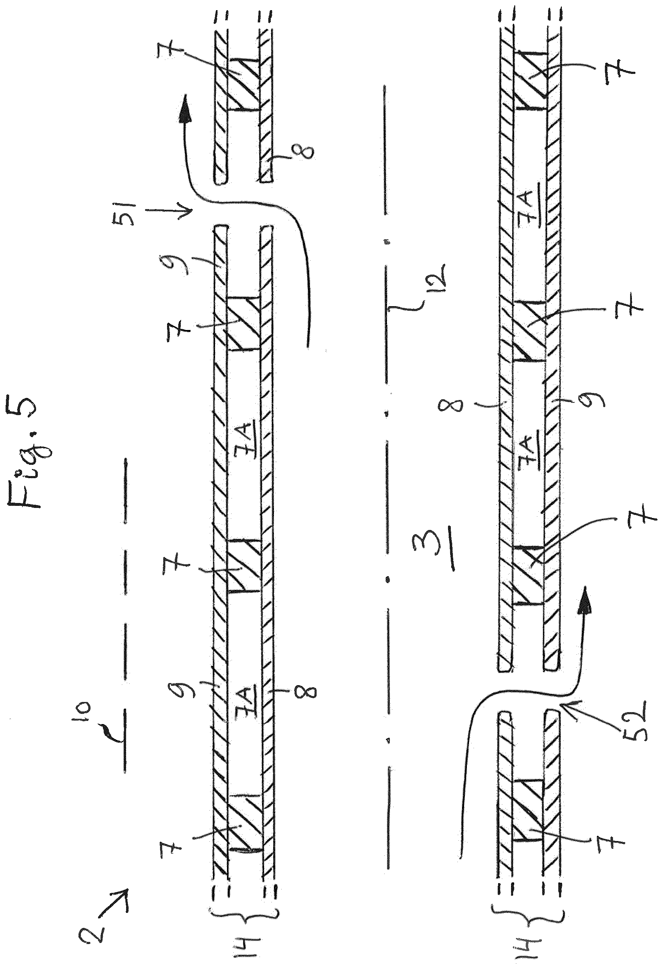

Two such perfusion side holes 51 and 52 of the randomized-skeleton wall section 14 are shown in Fig. 5. It is seen in Fig. 5 that each of the perfusion side holes 51, 52 is extending from the lumen structure 3, via successive interruptions of the inner liner 8, the randomized skeleton 7 and the outer laminate 9, to the radially outwards exterior environment of the tubular catheter wall 2 for enabling perfusion flow transversely through the tubular catheter wall 2 from the lumen structure 3 to said exterior environment. The perfusion flow has been indicated by two S-bend arrows. In radial view onto the imaginary "unrolled tube" state of the randomized skeleton 7 shown in Fig. 4, the perfusion side holes 51, 52 could for example have circular or oval shapes, wherein each perfusion side hole passes through a corresponding void of the cut pattern 7A. In other words, such a perfusion side hole does not intersect any of the struts 21L-28L, 31L-38L, 21R-28R and 31R-38R but passes between such struts.

The randomized skeleton of a catheter according to the invention may, for example, be designed as follows. As a starting point an initial "regular" skeleton design is taken, which comprises a plurality of interconnected "regular" struts having "regular" longitudinal strut edges (i.e. non-randomized strut edges), wherein the initial regular skeleton has been analysed by Finite Element Analysis (FEA) software to meet various design specifications (such as strength and stiffness properties) for the catheter to be designed. In a next "distortion" step of the skeleton design process, the initial regular skeleton is "distorted" by using a randomizing algorithm which transforms the regular longitudinal strut edges into longitudinal strut edges having random bends and/or random cusps (an example of such a randomizing algorithm is elucidated further below). The thus obtained randomized skeleton is then analysed by FEA software. If desired, this "distortion" step can be repeated several times to obtain different randomized skeleton designs to subject each of them to FEA. A chosen final design of a catheter according to the invention having such a randomized skeleton is analysed by FEA software in order to make sure that the randomized skeleton design having the random bends and/or the random cusps of the longitudinal strut edges meets various design specifications for the final catheter design.

Generally, as compared to a regular (i.e. non-randomized) skeleton, a corresponding randomized skeleton, obtained by randomizing starting-off from said regular skeleton, results in a randomized skeleton having large-scale strength and stiffness properties which are comparable to those of a regular non-randomized skeleton, but having small-scale bonding properties with the inner liner and the outer laminate which are better with the randomized skeleton, while also the catheter torquing properties are better with the randomized skeleton.

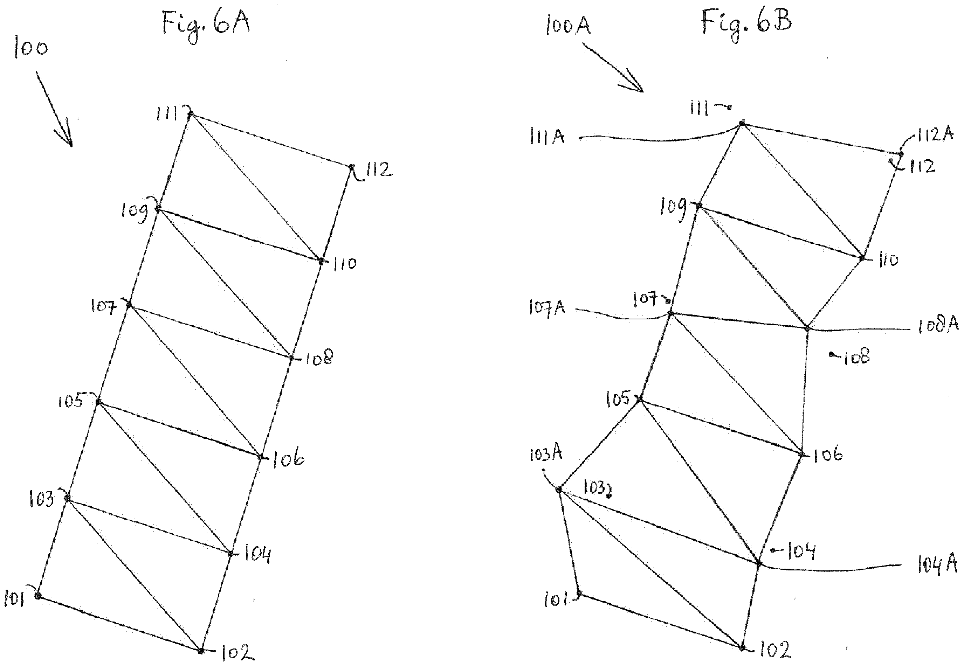

An example of the above-mentioned randomizing algorithm used in the above-mentioned example skeleton design process is now elucidated with reference to Figs. 6A-6B.

As seen in radial view on the randomized skeleton, each regular strut of an initial regular skeleton is modelled by an initial FEA mesh having an initial set of nodes. Fig. 6A shows the initial FEA mesh 100 of a portion of one regular strut of the initial regular skeleton, wherein the initial FEA mesh 100 has the nodes 101-112. In the distortion step of the skeleton design process some of these nodes 101-112 are each shifted in the longitudinal direction and/or the circumferential direction to obtain a distorted FEA mesh of a corresponding portion of a distorted strut. Others of these nodes 101-112 are not shifted at all in becoming part of said distorted FEA mesh. In the shown example of Figs. 6A-6B, the nodes 103, 104, 107, 108, 111 and 112 of the initial FEA mesh 100 of Fig. 6A are shifted in a randomized manner to become the distorted nodes 103A, 104A, 107A, 108A, 111A and 112A of the distorted FEA mesh 100A of Fig. 6B. The other nodes 101, 102, 105, 106, 109 and 110 of the initial FEA mesh 100 of Fig. 6A are the ones that are not shifted at all in becoming part of the distorted FEA mesh 100A.

The distorted FEA mesh 100A of Fig. 6B is only a very small portion of a larger distorted FEA mesh of a complete randomized skeleton 7 of a catheter according to the invention. Such a larger distorted FEA mesh is used in Finite Element Analysis of the randomized skeleton 7. The random shapes of all longitudinal strut edges 41, 42 of the randomized skeleton 7 can be determined by curve fitting relative to the nodes of such a larger distorted FEA mesh. So a portion of a longitudinal strut edge 41 may be the result of curve fitting relative to the nodes 101, 103A, 105, 107A, 109 and 111A of the distorted FEA mesh 100A of Fig. 6B, while a portion of a longitudinal strut edge 42 may be the result of curve fitting relative to the nodes 102, 104A, 106, 108A, 110 and 112A of the distorted FEA mesh 100A. Fig. 4 illustrates the results of such curve fitting for all left-handed struts 21L-28L, 31L-38L and for all right-handed struts 21R-28R, 31R-38R. It is seen that every strut of Fig. 4 has a unique shape (also see Fig. 2).

As mentioned, in the present example randomizing algorithm, the nodes 103, 104, 107, 108, 111 and 112 of the initial FEA mesh 100 of Fig. 6A have been shifted in a randomized manner to become the distorted nodes 103A, 104A, 107A, 108A, 111A and 112A of the distorted FEA mesh 100A of Fig. 6B. From Fig. 6B, which additionally shows the positions of the nodes 103, 104, 107, 108, 111 and 112, it can be seen that the distorted nodes 103A, 104A, 107A, 108A, 111A and 112A are the result of random shifting. In the shown example the randomizing is by a Normal distribution, which means that also the displacement distances involved in said shifting will have a Normal distribution for the huge numbers of nodes that have to be shifted for a complete randomized skeleton to be designed. It is possible to limit the maximum displacement distance and/or the minimum displacement distance involved in said shifting of the randomizing algorithm by pre-determined values. Generally, the randomizing algorithm can be based on randomizing by various other distribution types than said Normal distribution, such as for example Lognormal, Loglogistic or Weibull distributions.

The invention can be practiced with many various structures, many various materials, many various shapes and many various dimensions of the randomized skeleton, inner liner and outer laminate of the catheter, and with many various additional features and many various accessories of the catheter, such as the many various structures, the many various materials, the many various shapes and the many various dimensions of skeletons, inner liners and outer laminates, and the many various additional features and the many various accessories that are used in known catheters for delivering medical therapy to a remote site in a body, as long as the catheter is within the scope of the appended claims. Also, many various processes may be used for designing a randomized skeleton of a catheter according to the invention, and many various randomizing algorithms may be used therein.

Regarding the overall skeleton cut-away ratio the following general remark is made. If, everywhere in a longitudinally continuous longitudinal section of a catheter wall, the catheter wall has a totally uninterrupted tubular metal reinforcement wall, for example in the form of a totally uninterrupted metal hypotube, then said longitudinal section has an overall skeleton cut-away ratio of 0%.

In the shown example the randomized skeleton 7 has the following dimensions. The randomized-skeleton range 15 is extending between the distal end 5 and 150.0 mm proximal from the distal end 5. So the randomized skeleton 7 has a length of 150.0 mm in the longitudinal direction 10. The randomized skeleton 7 has an outer diameter of 1.651 mm and an inner diameter of 1.529 mm. It is noted that in the shown example, the randomized skeleton 7 ends at the distal end 5 of the catheter 1. Alternatively, however, the inner liner 8 and/or the outer laminate 9 may extend a bit distally beyond the randomized skeleton 7 to make the distal end 5 atraumatic.

It is further noted that according to the invention typical dimensions of some parts and aspects of the invention, and applicable practical ranges of such dimensions could be as follows in case the catheter is a microcatheter for interventional cardiovascular or neurovascular applications. Effective length of the catheter (as measured from the distal end of a hub of the catheter) can be in the range between 130 cm and 160 cm, and can typically be 135 cm. Maximum outer diameter of the randomized-skeleton wall section of the tubular catheter wall of the catheter can be in the range between 0.60 mm and 1.20 mm, and can typically be 0.75 mm, while noting that the word "maximum" in the term "maximum outer diameter" is referring to the maximum value as considered over the full length of the randomized-skeleton wall section. Minimum inner diameter of the randomized-skeleton wall section of the tubular catheter wall of the catheter can be in the range between 0.36 mm and 0.55 mm, and can typically be 0.45 mm, while noting that the word "minimum" in the term "minimum inner diameter" is referring to the minimum value as considered over the full length of the randomized-skeleton wall section. For example, the following typical combinations of maximum outer diameter and minimum inner diameter of the randomized-skeleton wall section are possible: maximum outer diameter 0.75 mm in combination with minimum inner diameter 0.45 mm, maximum outer diameter 1.20 mm in combination with minimum inner diameter 0.55 mm, or maximum outer diameter 0.60 mm in combination with minimum inner diameter 0.36 mm.

However, the present invention can be embodied in various other catheters for delivering medical therapy to a remote site in a body, which can be non-vascular and/or non-micro catheters, such as for example catheters for delivering medical therapy to renal vessels, fallopian tubes, and other such vessels and sites. In such cases typical dimensions of some parts and aspects of the invention, and applicable practical ranges of such dimensions may be different from those mentioned above for microcatheters for interventional cardiovascular or neurovascular applications.

## Claims

1. A catheter (1) for delivering medical therapy to a remote site in a body, wherein:
the catheter has a proximal end (4), a distal end (5) and a longitudinal direction (10) extending from the proximal end to the distal end;
the catheter comprises a tubular catheter wall (2), which is surrounding a lumen structure (3) of the catheter, and which is extending from the distal end (5), proximally along the longitudinal direction, to at least 100 mm proximal from the distal end;
the catheter wall (2) comprises a randomized-skeleton wall section (14), which is extending in a randomized-skeleton range (15) along the longitudinal direction, wherein said randomized-skeleton range is at least extending between 10 mm proximal from the distal end and 100 mm proximal from the distal end (5);
the randomized-skeleton wall section (14) comprises:
- a randomized skeleton (7), which is extending at least all along said randomized-skeleton range, and which provides reinforcement of the catheter wall (2) in the longitudinal direction (10), as well in a circumferential direction (11) around the longitudinal direction (10), wherein the randomized skeleton (7) is an interrupted tubular reinforcement wall obtained by microfabricating a cut pattern (7A) in a fully uninterrupted tubular reinforcement wall made of a reinforcement material,
- an inner liner (8), which is co-axially surrounded by said randomized skeleton (7) relative to a center line (12) of the catheter wall (2), and
- an outer laminate (9), which is co-axially surrounding said randomized skeleton (7) relative to the center line (12) of the catheter wall;
the randomized skeleton (7) comprises a plurality of interconnected struts (21L-28L, 31L-38L, 21R-28R, 31R-38R), wherein, as seen in radial view on the randomized skeleton, said radial view being radially in a cylindrical coordinate system relative to the center line (12):
- each of said struts (21L-28L, 31L-38L, 21R-28R, 31R-38R) has a corresponding pair of two mutually opposing longitudinal strut edges (41, 42) alongside one another, which are determining a corresponding distribution of tangential thickness of a strut concerned longitudinally along the strut concerned, said tangential thickness being tangentially in a cylindrical coordinate system relative to the center line (12),
- said longitudinal strut edges (41, 42) are bordering said cut pattern (7A), and
- each of said longitudinal strut edges (41, 42) has random bends and/or random cusps;
an overall skeleton cut-away ratio of a longitudinal section of the catheter wall (2) in a longitudinal range along the longitudinal direction (10) is defined as the percentage of:
- the volume of all parts, within said longitudinal range, of said reinforcement material being cut-away from said fully uninterrupted tubular reinforcement wall according to said microfabricated cut pattern (7A), relative to
- the volume of all parts, within said longitudinal range, of said reinforcement material of said fully uninterrupted tubular reinforcement wall; and
the overall skeleton cut-away ratio of the randomized-skeleton wall section (14) in said randomized-skeleton range (15) is between 40% and 90%.

2. The catheter (1) according to claim 1, wherein said randomized-skeleton range (15) is at least extending between 0.5 mm proximal from the distal end (5) and 100 mm proximal from the distal end (5).

3. The catheter (1) according to claim 2, wherein said randomized-skeleton range (15) is at least extending between the distal end (5) and 100 mm proximal from the distal end (5).

4. The catheter (1) according to any one of the preceding claims, wherein the overall skeleton cut-away ratio of the randomized-skeleton wall section (14) in said randomized-skeleton range (15) is between 60% and 85%.

5. The catheter (1) according to any one of the preceding claims, wherein a radiopaque element is embedded in the catheter wall (2) in that the radiopaque element is located in a space of the cut pattern (7A), said space being bounded by the randomized skeleton (7), the inner layer (8) and the outer laminate (9).

6. The catheter (1) according to any one of the preceding claims, wherein the catheter is a rapid exchange catheter having the tubular catheter wall (2) as a distal rapid exchange tubular segment which is spaced from the proximal end (4) of the rapid exchange catheter.

7. The catheter (1) according to claim 6, wherein the tubular catheter wall (2) being said distal rapid exchange tubular segment of said rapid exchange catheter comprises at least one perfusion side hole (51, 52), which is extending from the lumen structure (3), via successive interruptions of the inner liner (8), the randomized skeleton (7) and the outer laminate (9), to the radially outwards exterior environment of the tubular catheter wall (2) for enabling perfusion flow transversely through the tubular catheter wall (2) from the lumen structure (3) to said exterior environment.

8. The catheter (1) according to any one of claims 1-5, wherein the catheter is an over-the-wire (OTW) catheter, wherein the tubular catheter wall (2) and the lumen structure (3) of the catheter are extending all along the longitudinal direction (10) from the proximal end (4) to the distal end (5).

9. The catheter (1) according to claim 8, wherein the tubular catheter wall (2) of said over-the-wire (OTW) catheter comprises at least one perfusion side hole (51, 52), which is extending from the lumen structure (3), via successive interruptions of the inner liner (8), the randomized skeleton (7) and the outer laminate (9), to the radially outwards exterior environment of the tubular catheter wall (2) for enabling perfusion flow transversely through the tubular catheter wall (2) from the lumen structure (3) to said exterior environment.
